(19) Europäisches Patentamt / European Patent Office / Office européen des brevets

(11) **EP 4 106 712 B1**

(12) **EUROPEAN PATENT SPECIFICATION**

(45) Date of publication and mention
of the grant of the patent:
**07.01.2026 Bulletin 2026/02**

(21) Application number: **21705974.0**

(22) Date of filing: **19.02.2021**

(51) International Patent Classification (IPC):
*A61K 8/02* (2006.01)    *A61K 8/26* (2006.01)
*A61K 8/27* (2006.01)    *A61K 8/34* (2006.01)
*A61K 8/37* (2006.01)    *A61Q 1/12* (2006.01)

(52) Cooperative Patent Classification (CPC):
**A61K 8/022; A61K 8/26; A61K 8/27; A61K 8/342;
A61K 8/37; A61Q 1/12;** A61K 2800/805

(86) International application number:
**PCT/EP2021/054177**

(87) International publication number:
**WO 2021/165477 (26.08.2021 Gazette 2021/34)**

(54) **USE OF MICA IN PRESSED POWDER**

VERWENDUNG VON GLIMMER IN GEPRESSTEM PULVER

UTILISATION DE MICA EN POUDRE COMPRIMÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.02.2020 EP 20305166**

(43) Date of publication of application:
**28.12.2022 Bulletin 2022/52**

(73) Proprietor: **ImerTech SAS
75015 Paris (FR)**

(72) Inventors:
• **REMIA, Elodie
31300 Toulouse (FR)**
• **KARAGOZ, Céline
29900 Concarneau (FR)**

(74) Representative: **Haseltine Lake Kempner LLP
One Portwall Square
Portwall Lane
Bristol BS1 6BH (GB)**

(56) References cited:
**WO-A1-2016/146708    US-A1- 2009 041 698
US-B1- 6 348 536**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates generally to the use of mica having a lamellarity index of less than about 3.0 to enhance the cohesion of a pressed powder. The present invention further relates to pressed powders comprising mica having a lamellarity index of less than about 3.0, and methods for making said pressed powders.

### BACKGROUND

Inorganic particulate materials are commonly used as fillers in cosmetic products such as pressed powders. There is an ongoing need to develop new cosmetic products having enhanced properties.

WO 2016/146708 A1 is directed to cosmetic compositions comprising an inorganic particulate which is used in the cosmetic composition as a colour booster, cohesion enhancer, coverage enhance, opacity enhancer, and/or crack prevention additive,wherein the particulate has a lamellarity index of at least 1.0.

### SUMMARY

In accordance with a first embodiment of the present invention there is provided a use of mica having a lamellarity index of less than about 3.0 as a cohesion enhancer in a pressed powder.

In accordance with a second embodiment of the present invention there is provided a pressed powder comprising mica having a lamellarity index of less than about 3.0.

In accordance with a third embodiment of the present invention there is provided a method for making the pressed powder of the second embodiment of the present invention.

In accordance with a fourth embodiment of the present invention there is provided a method for enhancing the cohesion of a pressed powder, the method comprising incorporating mica having a lamellarity index of less than about 3.0 during manufacture of the pressed powder.

In accordance with a fifth embodiment of the present invention there is provided a use of the pressed powder of the second embodiment of the present invention in a cosmetic method of applying the pressed powder to the skin of a human.

In certain embodiments of any embodiment of the present invention, the mica has a lamellarity index of equal to or greater than about 0.5.

In certain embodiments of any embodiment of the present invention, the mica has a lamellarity index of greater than about 1.0.

In certain embodiments of any embodiment of the present invention, the mica is present in the pressed powder in an amount equal to or greater than about 50 wt%. For example, the mica may be present in the pressed powder in an amount equal to or greater than about 60 wt% or equal to or greater than about 70 wt% or equal to or greater than about 80 wt%.

In certain embodiments of any embodiment of the present invention, the pressed powder comprises equal to or less than about 10 wt% of a cohesive agent other than the mica. For example, the pressed powder may comprise equal to or less than about 5 wt% of a cohesive agent other than the mica.

Certain embodiments of any embodiment of the present invention may provide one or more of the following advantages:

- improved cohesion of a pressed powder (e.g. as demonstrated using a drop test);
- increased amount of mica in the pressed powder;
- decreased amount of other cohesive agents in the pressed powder;
- decreased amount of binder in the pressed powder.

The details, examples and preferences provided in relation to any particular one or more of the stated embodiments of the present invention will be further described herein and apply equally to all embodiments of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based on the surprising finding that mica having a low lamellarity index provides improved cohesion in a pressed powder compared to mica having a high lamellarity index. In particular, the present invention is based on the surprising finding that the use of mica having a lamellarity index of less than about 3.0 in a pressed powder provides good cohesion that is acceptable in a cosmetic application. This is particularly surprising since this effect has not been seen for talc, a particulate material that is currently commonly used in pressed powders, or for any other powdered cohesive agent. In some cases, it has been seen that, on the contrary, increasing the lamellarity index increases the cohesion of pressed powders.

The present invention is further based on the surprising finding that mica having a low lamellarity index (e.g. a lamellarity index of less than about 3.0) can be used in pressed powders in high amounts, for example equal to or greater than about 50 wt%. Still further, the present invention is based on the surprising finding that mica having a low lamellarity index (e.g. a lamellarity index of less than about 3.0) can be used in pressed powders to provide good cohesion that is acceptable in a cosmetic application in the presence of a reduced amount of other cohesive agents such as talc, bentonite, zinc stearate and magnesium stearate. For example, mica having a low lamellarity index (e.g. a lamellarity index of less than about 3.0) can be used in pressed powders comprising equal to or less than about 10 wt% of other cohesive agents, where the pressed powder still has good adhesion that is acceptable in a cosmetic application.

Mica is a group of sheet phyllosilicate monoclinic minerals having the general formula:

$$X_2Y_{4-6}Z_8O_{20}(OH, F)_4,$$

in which X is K, Na or Ca, or less commonly Ba, Rb or Cs; Y is Al, Mg or Fe, or less commonly Mn, Cr, Ti or Li; Z is Si or Al, or less commonly $Fe^{3+}$ or Ti.

Micas can be dioctahedral (Y = 4) or trioctahedral (Y = 6). If X is K or Na, the mica is common mica. If X is Ca, the mica is brittle mica.

Dioctahedral micas include muscovite. Trioctahedral micas include common micas such as biotite, lepidolite, phlogopite and zinnwaldite, and brittle micas such as clintonite.

Sericite is a very fine mica crystal obtained by alteration (generally hydrothermal) and can include a range of different minerals including a range of different micas such as paragonite and sodium micas, and other non-mica minerals such as quartz, feldspar and kaolinite. Crushed micas observed by SEM will generally have any shape (sometimes torn in appearance), whilst sericite will have the appearance of crystals (e.g. elongated hexagon, needle etc.).

In certain embodiments, the mica used in the present invention is not sericite.

Preferably, the mica used in the present invention comprises at least about 70 wt% muscovite. More preferably, the mica used in the present invention comprises at least about 75 wt% or at least about 80 wt% or at least about 85 wt% muscovite or at least about 90 wt% muscovite. For example, the mica used in the present invention may comprise up to about 100 wt% muscovite, for example up to about 98 wt% or up to about 95 wt% or up to about 92 wt% muscovite.

The muscovite has the advantage of being luminous and transparent, which is particularly interesting for the pressed powders, for example the foundations. On the contrary, sericite is mat and covering, whereas phlogopite is not luminous.

The mica may, for example, be obtained from a natural source by grinding. The mica may be obtained by crushing and then grinding a mineral source, which may be followed by a particle size classification step, in order to obtain a product having a desired particle size distribution. The particulate solid material may be ground autogenously, i.e. by attrition between the particles of the solid material themselves, or, alternatively, in the presence of a particulate grinding medium comprising particles of a different material from the mica to be ground. These processes may be carried out with or without the presence of a dispersant and biocides, which may be added at any stage of the process.

The mica may be prepared using techniques well known to a person of skill in the art, for example, techniques selected from comminution (e.g., crushing, grinding, milling), classification (e.g., hydrodynamic selection, screening and/or sieving) and drying.

The mica used in the present invention has a lamellarity index of less than about 3.0. Preferably, the mica used in the present invention may have a lamellarity index equal to or less than about 2.8, more preferably equal to or less than about 2.6 or equal to or less than about 2.5 or equal to or less than about 2.4 or equal to or less than about 2.2 or equal to or less than about 2.0 or equal to or less than about 1.8 or equal to or less than about 1.6 or equal to or less than about 1.5.

Preferably, the mica used in the present invention has a lamellarity index equal to or greater than about 0.5. More preferably, the mica used in the present invention has a lamellarity index greater than about 1.0. Even more preferably, the mica have a lamellarity index equal to or greater than about 0.6 or equal to or greater than about 0.8 or equal to or greater than about 1.0 or equal to or greater than about 1.2 or equal to or greater than about 1.4 or equal to or greater than about 1.5 or equal to or greater than about 1.6 or equal to or greater than about 1.8 or equal to or greater than about 2.0.

Preferably, the mica used in the present invention has a lamellarity index ranging from about 0.5 to less than about 3.0.

More preferably, the mica used in the present invention has a lamellarity index ranging from greater than about 1.0 to less than about 3.0.

Even more preferably, the mica used in the present invention has a lamellarity index ranging from about 1.2 to about 2.8 or from about 1.2 to about 2.5 or from about 1.2 to about 2.2 or from about 1.2 to about 2.0 or from about 1.2 to about 1.8 or from about 1.2 to about 1.5.

As used herein, the term "lamellarity index" is defined by the following ratio:

$$\frac{d_{50laser} - d_{50sedi}}{d_{50sedi}}$$

in which "$d_{50laser}$" is the value of the mean particle size ($d_{50}$) obtained by a particle size measurement by Malvern laser scattering (standard ISO 13320-1) and "$d_{50sedi}$" is the value of the median diameter obtained by sedimentation using a sedigraph (standard ISO 13317-3), as described below. Reference may be made to the article by G. Baudet and J. P. Rona, Ind. Min. Mines et Carr. Les techn. June, July 1990, pp 55-61, which shows that this index is correlated to the mean ratio of the largest dimension of the particle to its smallest dimension.

In the sedimentation technique referred to above, particle size properties referred to herein for the particulate materials are as measured in a well-known manner by sedimentation of the particulate material in a fully dispersed condition in an aqueous medium using a Sedigraph III+ 5125 machine as supplied by Micromeritics Instruments Corporation, Norcross, Georgia, USA (www.micromeritics.com), referred to herein as a "Micromeritics Sedigraph III+ 5125 unit", and based on application of Stokes' Law. Such a machine provides measurements and a plot of the cumulative percentage by weight of particles having a size, referred to in the art as the 'equivalent spherical diameter' (e.s.d), less than given e.s.d values. The mean particle size d50 is the value determined in this way of the particle e.s.d at which there are 50% by weight of the particles which have an equivalent spherical diameter less than that d50 value. The d95 value is the value at which 95% by weight of the particles have an esd less than that d95 value. Particle size properties is determined in accordance with ISO 13317-3, or any method equivalent thereto. Preferably, 4,8g of the mineral are first added in a beaker to 80 mL of a dispersing solution (the dispersing solution being prepared as: 250 mg of Calgon (sodium metaphosphate ; CAS number 68915-31-1) and 1 ml of Triton X100 (Polyethylene glycol tert-octylphenyl ether ; CAS number 9002-93-1) dissolved in a litre of demineralised water). The beaker is then placed in an ultra-sonic bath during 600 seconds to remove air bubbles. The resulting suspension is then analysed in a Sedigraph III+ 5125 machine.

In the Malvern laser light scattering technique referred to above, the size of particles in powders, suspensions and emulsions may be measured using the diffraction of a laser beam, based on an application of Mie theory. Such a machine, for example a Malvern Mastersizer 2000 (as supplied by Malvern Instruments) provides measurements and a plot of the cumulative percentage by volume of particles having a size, referred to in the art as the 'equivalent spherical diameter' (e.s.d), less than given e.s.d values. The mean particle size $d_{50}$ is the value determined in this way of the particle e.s.d at which there are 50% by weight of the particles which have an equivalent spherical diameter less than that $d_{50}$ value. Particle size properties is determined in accordance with ISO 13320-1, or any method equivalent thereto. For the avoidance of doubt, the measurement of particle size using laser light scattering is not an equivalent method to the sedimentation method referred to above.

For example, 1g of the mineral is first added in a beaker and then 2 to 2.5 ml of ethanol are added onto the powder. The suspension is then mixed with a manual stirrer. The beaker is then placed in an ultra-sonic bath during 30 seconds to remove air bubbles. The resulting suspension is then analysed in a Mastersizer 2000 machine, applying the **Mie theory.**

Preferably, the mica has a $d_{50laser}$ equal to or less than about 40.0 $\mu$m. More preferably, the mica has a $d_{50laser}$ equal to or less than about 35.0 $\mu$m or equal to or less than about 30.0 $\mu$m or equal to or less than about 25.0 $\mu$m or equal to or less than about 20.0 $\mu$m. Even more preferably, the mica has a $d_{50laser}$ equal to or less than about 20.0 $\mu$m. For example, the mica has a $d_{50laser}$ equal to or less than about 19.0 $\mu$m or equal to or less than about 18.0 $\mu$m or equal to or less than about 17.0 $\mu$m or equal to or less than about 16.0 $\mu$m or equal to or less than about 15.0 $\mu$m or equal to or less than about 14.0 $\mu$m or equal to or less than about 13.0 $\mu$m or equal to or less than about 12.0 $\mu$m or equal to or less than about 11.0 $\mu$m or equal to or less than about 10.0 $\mu$m.

Preferably the mica has a $d_{50laser}$ equal to or greater than about 3.0 $\mu$m. More preferably, the mica has a $d_{50laser}$ equal to or greater than about 4.0 $\mu$m or equal to or greater than about 5.0 $\mu$m or equal to or greater than about 6.0 $\mu$m or equal to or greater than about 7.0 $\mu$m or equal to or greater than about 8.0 $\mu$m. Even more preferably the mica has a $d_{50laser}$ equal to or greater than about 8.0 $\mu$m. For example, the mica has a $d_{50laser}$ equal to or greater than about 9.0 $\mu$m or equal to or greater than about 10.0 $\mu$m or equal to or greater than about 11.0 $\mu$m or equal to or greater than about 12.0 $\mu$m or equal to or greater than about 13.0 $\mu$m or equal to or greater than about 14.0 $\mu$m or equal to or greater than about 15.0 $\mu$m.

Preferably, the mica has a $d_{50laser}$ ranging from about 3.0 $\mu$m to about 40.0 $\mu$m or from about 5.0 $\mu$m to about 30.0 $\mu$m or from about 8.0 $\mu$m to about 20.0 $\mu$m or from about 10.0 $\mu$m to about 20.0 $\mu$m or from about 8.0 $\mu$m to about 18.0 $\mu$m or from about 8.0 $\mu$m to about 12.0 $\mu$m. It is interesting to have such a $d_{50laser}$ because it provides good sensory properties.

Preferably, the mica has a $d_{50sedi}$ equal to or less than about 20.0 $\mu$m. More preferably, the mica has a $d_{50sedi}$ equal to or less than about 15.0 $\mu$m or equal to or less than about 10.0 $\mu$m or equal to or less than about 8.0 $\mu$m. Even more preferably, the mica has a $d_{50sedi}$ equal to or less than about 8.0 $\mu$m. For example, the mica has a $d_{50sedi}$ equal to or less than about 7.5 $\mu$m or equal to or less than about 7.0 $\mu$m or equal to or less than about 6.5 $\mu$m or equal to or less than about 6.0 $\mu$m or equal to or less than about 5.5 $\mu$m or equal to or less than about 5.0 $\mu$m or equal to or less than about 4.5 $\mu$m.

**Preferably** the mica has a $d_{50sedi}$ equal to or greater than about 0.5 $\mu$m. More preferably, the mica has a $d_{50sedi}$ equal to or greater than about 1.0 $\mu$m or equal to or greater than about 1.5 $\mu$m or equal to or greater than about 2.0 $\mu$m or equal to or greater than about 2.5 $\mu$m or equal to or greater than about 3.0 $\mu$m or equal to or greater than about 3.5 $\mu$m or equal to or greater than about 4.0 $\mu$m.

Preferably, the mica has a $d_{50sedi}$ ranging from about 0.5 $\mu$m to about 20.0 $\mu$m or from about 1.0 $\mu$m to about 10.0 $\mu$m or from about 2.0 $\mu$m to about 8.0 $\mu$m or from about 3.0 $\mu$m to about 7.0 $\mu$m or from about 3.0 $\mu$m to about 6.5 $\mu$m or from about 3.5 $\mu$m to about 5.0 $\mu$m.

In certain embodiments, the mica has a lamellarity index equal to or greater than about 2.0 and less than about 3.0, a $d_{50laser}$ ranging from about 14.0 $\mu$m to about 18.0 $\mu$m and a $d_{50sedi}$ ranging from about 3.0 $\mu$m to about 6.0 $\mu$m.

In certain embodiments, the mica has a lamellarity index equal to or greater than about 1.0 and equal to or less than about 2.0, a $d_{50laser}$ ranging from about 8.0 $\mu$m to about 12.0 $\mu$m and a $d_{50sedi}$ ranging from about 3.5 $\mu$m to about 5.0 $\mu$m.

In certain embodiments, the mica has a lamellarity index equal to or greater than about 1.5 and equal to or less than about 2.5, a $d_{50laser}$ ranging from about 15.0 $\mu$m to about 18.0 $\mu$m and a $d_{50sedi}$ ranging from about 5.0 $\mu$m to about 7.0 $\mu$m.

In certain embodiments, the mica has a lamellarity index equal to or greater than about 1.5 and equal to or less than about 2.5, a $d_{50laser}$ ranging from about 11.0 $\mu$m to about 13.0 $\mu$m and a $d_{50sedi}$ ranging from about 3.0 $\mu$m to about 5.0 $\mu$m.

In certain embodiments, the mica has a lamellarity index equal to or greater than about 1.5 and equal to or less than about 2.5, a $d_{50laser}$ ranging from about 17.0 $\mu$m to about 19.0 $\mu$m and a $d_{50sedi}$ ranging from about 5.0 $\mu$m to about 7.0 $\mu$m.

Preferably, the mica has a $d_{90laser}$ equal to or greater than about 20 $\mu$m. More preferably, the mica has a $d_{90laser}$ equal to or greater than about 25 $\mu$m or equal to or greater than about 30 $\mu$m or equal to or greater than about 35 $\mu$m.

Preferably, the mica has a $d_{90laser}$ equal to or less than about 70 $\mu$m. More preferably, the mica has a $d_{90laser}$ equal to or less than about 65 $\mu$m or equal to or less than about 60 $\mu$m or equal to or less than about 55 $\mu$m or equal to or less than about 50 $\mu$m.

Preferably, the mica has a $d_{90laser}$ ranging from about 20 $\mu$m to about 70 $\mu$m or from about 30 $\mu$m to about 60 $\mu$m or from about 35 $\mu$m to about 55 $\mu$m.

Preferably, the mica had a $d_{10laser}$ equal to or greater than about 3.0 $\mu$m. More preferably, the mica has a $d_{10laser}$ equal to or greater than about 3.5 $\mu$m or equal to or greater than about 4.0 $\mu$m.

Preferably, the mica has a $d_{10laser}$ equal to or less than about 7.0 $\mu$m. More preferably, the mica has a $d_{10laser}$ equal to or less than about 6.5 $\mu$m or equal to or less than about 6.0 $\mu$m or equal to or less than about 5.5 $\mu$m.

Preferably, the mica has a $d_{10laser}$ ranging from about 3.0 $\mu$m to about 7.0 $\mu$m or from about 3.5 $\mu$m to about 6.5 $\mu$m or from about 4.0 $\mu$m to about 6.0 $\mu$m.

Preferably, the mica has a BET surface area equal to or greater than about 3 $m^2$/g. More preferably, the mica has a BET surface area equal to or greater than about 4 $m^2$/g or equal to or greater than about 5 $m^2$/g or equal to or greater than about 6 $m^2$/g or equal to or greater than about 7 $m^2$/g.

Preferably, the mica has a BET surface area equal to or less than about 15 $m^2$/g. More preferably, the mica has a BET surface area equal to or less than about 14 $m^2$/g or equal to or less than about 13 $m^2$/g or equal to or less than about 12 $m^2$/g or equal to or less than about 11 $m^2$/g or equal to or less than about 10 $m^2$/g.

Preferably, the mica has a BET surface area ranging from about 3 $m^2$/g to about 15 $m^2$/g or from about 5 $m^2$/g to about 12 $m^2$/g or from about 6 $m^2$/g to about 10 $m^2$/g.

As used herein, "specific surface area (BET)" means the area of the surface of the particles of the particulate with respect to unit mass, determined according to the BET method by the quantity of nitrogen adsorbed on the surface of said particles so to as to form a monomolecular layer completely covering said surface (measurement according to the BET method, AFNOR standard X11-621 and 622 or ISO 9277). In certain embodiments, specific surface area is determined in accordance with ISO 9277, or any method equivalent thereto.

Preferably, the mica has an oil absorption equal to or greater than about 60 %. More preferably, the mica has an oil absorption equal to or greater than about 65 % or equal to or greater than about 70 % or equal to or greater than about 75 % or equal to or greater than about 80 %.

Preferably, the mica has an oil absorption equal to or less than about 100 %. More preferably, the mica may have an oil absorption equal to or less than about 95 % or equal to or less than about 90 %.

Preferably, the mica has an oil absorption ranging from about 60 % to about 100 % or from about 70 % to about 90 %.

Oil absorption (i.e., amount of oil absorbed per amount of mica, e.g., ml or g of oil per 100 g of particulate) may be determined by any suitable method, for example, ASTM D1483.

In certain embodiments, the mica is not surface treated with silicone. In certain embodiments, the mica is not coated by titanium. In certain embodiments, the mica is not coated by colorant.

Preferably, the mica is uncoated. The use of uncoated mica may exclude micas that are used as pigments. This may be particularly advantageous in that the mica used in the pressed powder can be considered to be a natural product.

The mica described herein is suitable for and/or intended for use in a pressed powder. A pressed powder is a dry, bulk solid made up of particles that are compacted together so that they do not flow freely when shaken or titled. By "dry", it is meant that the pressed powder comprises equal to or less than about 5.0 wt% moisture, for example equal to or less than about 4.5 wt% or equal to or less than about 4.0 wt% or equal to or less than about 3.5 wt% or equal to or less than about 3.0 wt% or equal to or less than about 2.5 wt% or equal to or less than about 2.0 wt% or equal to or less than about 1.5 wt% or equal to or less than about 1.0 wt% moisture. This may be measured by heating the pressed powder until its weight does not change and determining the difference in weight.

In certain embodiments, the pressed powder comprises equal to or less than about 5.0 wt% moisture.

The pressed powders described herein are particularly cosmetics. The pressed powders described herein may, for example, be used in a method of applying the pressed powder to the skin of a human.

As used herein, the term "cosmetic" means a product intended to be applied to the human body for beautifying, promoting attractiveness, or altering the appearance without affecting the body's structure or functions. In certain embodiments, the cosmetic is a decorative cosmetic. In particular, the term cosmetic excludes products that can be used for a method of treatment of the human or animal body.

The pressed powder may, for example, be a primer, a concealer, a foundation, a blush, a bronzer, an eye shadow, a contour powder, a face powder, a highlighter, or an eyebrow powder.

The pressed powder may be prepared by any suitable or conventional method well known to those skilled in the art. Such methods generally comprise combining the components of the pressed powder in a liquid, slurry or solid form, mixing the components, optionally milling the mixture of components, and then forming the pressed powder therefrom. The components may be brought together in a blender or other mixing apparatus under conditions of suitably low shear so as to preserve the inherent properties of the particulate material. Forming may comprise drying and/or pressing, depending on the nature of the method of manufacture and the final form of the pressed powder.

The pressed powder may, for example, be made by a wet processing method or by a dry processing method. Generally, pressed powders are made by filling a mould with the pressed powder composition and applying pressure to compact the particles together. Various temperatures and pressures may be applied depending on the particular product. The application of pressure may be repeated numerous times to obtain the desired product. In wet processing methods, the particles of the pressed powder are present in a liquid solvent or suspension (e.g. an aqueous slurry) and the application of heat and/or pressure removes the liquid. In dry processing methods, no liquid is present.

The micas described herein may be used as cohesion enhancers in pressed powders. In other words, the micas described herein can be used to enhance the cohesion of a pressed powder compared to a pressed powder in which the mica is not present. The cohesion generally relates to the pressability of the pressed powder. An increase in cohesion may be determined by measuring the fracture resistance of a pressed powder. A suitable drop test method for determining fracture resistance is described in the Examples below. Enhanced cohesion enables process improvement and better handling.

In particular, the drop test is as described: to measure the cohesion of a formulation, three dishes filled with the pressed powder formulation are dropped in an upright position inside a cylinder onto a plate from a distance of 30 cm. This is repeated until the pressed powder formulation cracks. The number of drops taken to crack the pressed powder is recorded.

The drop test may be performed with a Drop test equipment by Cosmatic machines of Marchesini group.

A pressed powdered is considered to be cohesive if the drop test result is that the pressed powder shows the first cracks after at least 5 drops.

The pressed powders described herein may comprise equal to or greater than about 50 wt% of the mica described herein (i.e. mica having a lamellarity index of less than about 3.0 including all embodiments thereof). The pressed powder may, for example, comprise equal to or greater than about 55 wt% or equal to or greater than about 60 wt% or equal to or greater than about 65 wt% or equal to or greater than about 70 wt% or equal to or greater than about 75 wt% or equal to or greater than about 80 wt% of the mica described herein.

The pressed powders described herein may comprise equal to or less than about 90 wt% of the mica described herein (i.e. mica having a lamellarity index of less than about 3.0 including all embodiments thereof). The pressed powder may, for example, comprise equal to or less than about 88 wt% or equal to or less than about 86 wt% or equal to or less than about 85 wt% of the mica described herein.

For example, the pressed powder may comprise from about 50 wt% to about 90 wt% or from about 60 wt% to about 88 wt% or from about 70 wt% to about 86 wt% or from about 75 wt% to about 85 wt% or from about 80 wt% to about 85 wt% of the mica having a lamellarity index of less than about 3.0.

The pressed powders described herein may comprise equal to or less than about 10.0 wt% of other cohesive agents. For example, the pressed powder may comprise equal to or less than about 9.5 wt% or equal to or less than about 9.0 wt% or equal to or less than about 8.5 wt% or equal to or less than about 8.0 wt% or equal to or less than about 7.5 wt% or equal to or less than about 7.0 wt% of other cohesive agents.

The pressed powders described herein may comprise equal to or greater than about 0.5 wt% of other cohesive agents. For example, the pressed powder may comprise equal to or greater than about 1.0 wt% or equal to or greater than about 1.5 wt% or equal to or greater than about 2.0 wt% of other cohesive agents.

For example, the pressed powder may comprise from about 0 wt% to about 10.0 wt% or from about 0.5 wt% to about 9.0 wt% or from about 1.0 wt% to about 8.0 wt% of other cohesive agents.

By "other cohesive agents" it is meant powdered materials other than the mica having a lamellarity index of less than about 3.0 that act to enhance the cohesion of the pressed powder. Examples of other cohesive agents are talc, bentonite, magnesium stearate and zinc stearate. This does not include liquid binders.

The pressed powder may further comprise one or more colourant(s) and/or one or more binder(s) and/or one or more cosmetically acceptable base(s) in addition to the mica having a lamellarity index of less than about 3.0. In certain embodiments, the binder, when present, may be a constituent of the cosmetically acceptable base. In certain embodiments, the pressed powder comprises one or more colourant(s) and one or more binder(s) in addition to the mica.

The colourant (i.e., a component which imparts colour) may be an organic colourant and/or an inorganic colourant. Colourants for cosmetics are many and various. A list of colorant agents permitted for use in cosmetic products is provided in Annex IV to the Cosmetics Directive 76/768/EEC. Organic colourants include dyes and the like. Examples of organic colourants include species characterized in one of the following groups: indigoid, xanthenes, azo, nitro, triphenylmethane, quinoline and anthraquinone. Inorganic colourants include pigments, such as mineral pigments. In certain embodiments, the colourant is a mineral pigment, for example, one or more of zinc oxide, titanium dioxide, iron oxide (black, red, orange, yellow and/or brown), tin oxide, chrome oxide, ultramarine (blue, pink and/or violet), manganese violet (ammonium manganese (III) pyrophosphate) and Prussian blue (ferric ferrocynanide). In certain embodiments, the colourant is a pigment. In certain embodiments, the colourant is a mineral pigment. The colourant, for example, the mineral pigment or combinations thereof, may be selected depending on the desired colour for the cosmetic. In certain embodiments, the colourant may be nacre or a derivative thereof, providing a desirable pearlescence (also known as luster) and/or brilliance.

The colourant may constitute up to about 30.0 % by weight of the pressed powder, for example, up to about 25.0 % by weight, or up to about 20.0 % by weight of the pressed powder, for example, from about 0 % to about 20.0 % by weight, or from about 0.01 % to about 20.0 % by weight, or from about 0.1 % to about 20.0 % by weight, or from about 1.0% to about 20.0 % by weight, or from about 1.0 % to about 15.0 % by weight, or from about 2.0 % to about 15.0 % by weight, or from about 5.0 % to about 15.0 % by weight, or from about 7.5 % by weight to about 12.5 % by weight, or from about 4.0 % to about 10.0 % by weight of the pressed powder.

Where the mica having a lamellarity index of less than about 3.0 is present in the pressed powder in an amount equal to or greater than about 50.0 wt% and/or equal to or less than about 90.0 wt%, the colourant may be present in the pressed powder in an amount equal to or less than about 50.0 wt%. For example, the colourant may be present in the pressed powder in an amount equal to or less than about 40.0 wt% or equal to or less than about 30.0 wt% or equal to or less than about 20.0 wt% or equal to or less than about 10.0 wt% or equal to or less than about 7.5 wt% or equal to or less than about 5.0 wt%.

Where the mica having a lamellarity index of less than about 3.0 is present in the pressed powder in an amount equal to or greater than about 50.0 wt% and/or equal to or less than about 90.0 wt%, the colourant may be present in the pressed powder in an amount equal to or greater than about 0.5 wt%. For example, the colourant may be present in the pressed powder in an amount equal to or greater than about 1.0 wt% or equal to or greater than about 1.5 wt% or equal to or greater than about 2.0 wt% or equal to or greater than about 2.5 wt% or equal to or greater than about 3.0 wt% or equal to or greater than about 3.5 wt% or equal to or greater than about 4.0 wt% or equal to or greater than about 4.5 wt% or equal to or greater than about 5.0 wt%.

For example, the colourant may be present in the pressed powder in an amount ranging from about 0.5 wt% to about 50.0 wt% or from about 0.5 wt% to about 40.0 wt% or from about 0.5 wt% to about 30.0 wt% or from about 0.5 wt% to about 20.0 wt% or from about 0.5 wt% to about 10.0 wt% or from about 0.5 wt% to about 7.5 wt% or from about 0.5 wt% to about 5.0 wt%.

When present, the cosmetically acceptable base may be any base suitable for the intended purpose. In certain embodiments, the base is an oil and/or wax containing material. The base and, thus, the pressed powder, may comprise other components such as humectants, preservative, emollient, fragrance and antioxidant.

The binder, when present, may be a liquid binder. In certain embodiments, the binder is a liquid binder, for example, on oil-based binder. In certain embodiments, the liquid binder is a fatty acid or ester or salt thereof, or a combination of fatty acids and/or ester and/or salts thereof. In certain embodiments, the fatty acid or ester or salt thereof, or combinations thereof, is derived from vegetable oil, for example, coconut oil, palm oil, palm kernel oil soybean oil, corn oil, rapeseed oil, and the like. In certain embodiments, the binder is cocoate ester, for example, isoamyl cocoate. Other binders include silicone.

Suitable binder materials include polyhydric alcohol, hyaluronic acid and its salts, an amino acid and its salts, chondroitin sulfuric acid and its salts, lactic acid and its salts, pyroglutamic acid and its salts, uric acid and its salts, and mixtures thereof. Polyhydric alcohols include glycerin, diglycerin, triglycerin, ethylene glycol, diethylene glycol, propylene glycol, dipropylene glycol, hexylene glycol, 1,3-butylene glycol, 1,4-butylene glycol, glucose, maltose, sucrose, xylitose, sorbitol, maltitol, malbit, panthenol, hyaluronic acid and its salts, and mixtures thereof.

Further non-limiting examples of suitable binder materials are polyglycerin fatty acid esters, propylene glycol fatty acid esters, glycerin fatty acid esters, sorbitan fatty acid esters, sugar fatty acid esters, polyoxyethylene sorbitan fatty acid esters, polyoxyethylene sorbit fatty acid esters, polyethylene glycol fatty acid esters, polyoxyethylene castor oils, polyoxyethylene hardened castor oils, polyoxyethylene alkyl ethers, polyoxyethylene phytosterols, polyoxyethylene polyoxypropylene alkyl ethers, polyoxyethylene alkyl phenyl ethers, polyoxyethylene lanolins, polyoxyethylene lanolin alcohols, polyoxyethylene beeswax derivatives, polyoxyethylene fatty acid amides, and polyether silicone derivatives.

The fatty acids making the esters above can be saturated or unsaturated, straight or branched, and include those of natural origin having about 16-18 carbons. Non-limiting examples include triglyceryl beeswax, triglyceryl cetyl ether, tetraglyceryl cocoate, triglyceryl decyltetradecanol, diglyceryl diisostearate, triglyceryl diisostearate, decaglyceryl diisostearate, diglyceryl dioleate, triglyceryl dioleate, hexaglyceryl dioleate, decaglyceryl dioleate, triglyceryl distearate, hexaglyceryl distearate, decaglyceryl distearate, decaglyceryl trioleate, decaglyceryl heptaoleate, decaglyceryl heptastearate, hexaglyceryl hexaoleate, diglyceryl isostearate, tetraglyceryl isostearate, hexaglyceryl monoisostearate, diglyceryl lanolin alcohol ether, tetraglyceryl lauryl ether, diglyceryl oleate, triglyceryl oleate, tetraglyceryl oleate, hexaglyceryl oleate, diglyceryl oleyl ether, tetraglyceryl oleyl ether, diglyceryl sesquiisostearate, and diglyceryl sesquioleate and mixtures thereof.

Also suitable as binder materials are liquid paraffin, squalane, liquid petrolatum, mineral oil, and liquid polybutene.

Also suitable are natural oils which are typically a mixture of saturated and unsaturated fatty acid. Non-limiting examples of natural oil derived from plants include almond oil, olive oil, sesame oil, safflower oil, avocado oil, cottonseed oil, jojoba oil, castor bean oil, castor oil, rapeseed oil, soybean oil, palm kernel oil, coconut oil, hydrogenated vegetable oil, and cocoa butter. Non-limiting examples of natural oil derived from animal sources include mink oil and egg yolk oil.

Non-limiting examples of fatty alcohol which may be employed as binder are isostearyl alcohol, lanolin alcohol, oleyl alcohol, hexadecyl alcohol, octyldodecanol alcohol, linoleyl alcohol, linolenyl alcohol, and arachidyl alcohol.

Fatty acid can be natural or synthetic, saturated, unsaturated, linear, or branched. Non-limiting examples of fatty acid are adipic, caprylic, capric, isostearic, linoleic, ricinoleic, oleic, elaidic and erucic acid.

Non-limiting examples of fatty acid ester are cetyl ricinoleate, cetyl oleate, cetyl octanoate, cetyl acetate, glyceryl trioctanoate, isopropyl lanolate, isopropyl linoleate, isopropyl myristate, isopropyl palmitate, isopropyl oleate, isopropyl stearate, ethyl lactate, ethyl glutamate, ethyl laurate, ethyl linoleate, ethyl methacrylate, ethyl myristate, ethyl palmitate, diisopropyl adipate, octyl dodecyl myristate, octyl palmitate, octyl isopelargonate, octyl dodecyl lactate, tridecyl isononanoate, isotridecyl isononanoate, hexadecyl stearate, oleyl oleate, isononyl isononanoate, isostearyl myristate, dipenta-erythrytol ester, neopentyl glycol dioctanoate, and di(capryl/capric acid) propylene glycol and mixtures thereof. Other suitable esters include triglycerides such as caprylic triglycerides, capric triglyceride, isostearic triglyceride, adipic triglyceride and cholesterol derivatives such as cholesteryl oleate.

Non-volatile, straight, and branched silicone oil such as dimethicone and phenyl dimethicone is also useful.

The binder (e.g. the liquid binder) may constitute up to about 30.0 % by weight of the pressed powder, for example, from about 1.0 % to about 30.0 % by weight, or from about 1.0 % to about 25.0 % by weight, or from about 2.0 % to about 20.0 % by weight, or from about 2.0 % to about 15.0 % by weight, or from about 2.0 to about 10.0 % by weight, or from about 2.0 to about 7.5 % by weight of the pressed powder. It is interesting to have a reduced amount of binder so as to lower the cost of production of the pressed powder.

In an embodiment, the pressed powder comprises up to about 30.0 % by weight binder.

When the mica having a lamellarity index of less than about 3.0 is present in the pressed powder in an amount equal to or greater than about 50.0 wt% and/or equal to or less than about 90.0 wt%, the binder may be present in the pressed powder in an amount equal to or less than about 50.0 wt%. For example, the binder may be present in the pressed powder in an amount equal to or less than about 40.0 wt% or equal to or less than about 30.0 wt% or equal to or less than about 20.0 wt% or equal to or less than about 10.0 wt% or equal to or less than about 7.5 wt% or equal to or less than about 5.0 wt%.

Where the mica having a lamellarity index of less than about 3.0 is present in the pressed powder in an amount equal to or greater than about 50.0 wt% and/or equal to or less than about 90.0 wt%, the binder may be present in the pressed powder in an amount equal to or greater than about 0.5 wt%. For example, the binder may be present in the pressed powder in an amount equal to or greater than about 1.0 wt% or equal to or greater than about 1.5 wt% or equal to or greater than about 2.0 wt% or equal to or greater than about 2.5 wt% or equal to or greater than about 3.0 wt% or equal to or greater than about 3.5 wt% or equal to or greater than about 4.0 wt% or equal to or greater than about 4.5 wt% or equal to or greater than about 5.0 wt%.

For example, the binder may be present in the pressed powder in an amount ranging from about 0.5 wt% to about 50.0 wt% or from about 1.0 wt% to about 40.0 wt% or from about 2.0 wt% to about 30.0 wt% or from about 3.0 wt% to about 20.0 wt% or from about 4.0 wt% to about 10.0 wt% or from about 5.0 wt% to about 10.0 wt% or from about 5.0 wt% to about 7.5 wt%.

In certain embodiments, the pressed powder comprises from about 70.0 wt% to about 90.0 wt% of the mica having a lamellarity index of less than about 3.0, from about 0.5 wt% to about 7.5 wt% of a colourant, and from about 5.0 wt% to about 20.0 wt% of a binder. In certain embodiments, the pressed powder comprises from about 70.0 wt% to about 85.0 wt% of the mica having a lamellarity index of less than about 3.0, from about 0.5 wt% to about 7.5 wt% of a colourant, and from about 5.0 wt% to about 20.0 wt% of a binder. In such embodiments, the pressed powder may further comprise suitable amounts of one or more of humectants, preservative, emollient, fragrance and antioxidant, for example, up to about 10.0 % by weight so such components, based on the total weight of the composition, or up to about 5.0 % by weight of such components, or from about 0.001 % to about 2.5 % by weight of such components. In certain embodiments, the pressed powder is free of components other than the mica, colourant and binder.

EXAMPLES

*Example 1*

The particulate minerals described in Table 1 below were used to prepare a series of pressed powder compacts, as described in more detail below.

Table 1.

| Mineral | $d_{50laser}$ ($\mu$m) | $d_{50sedi}$ ($\mu$m) | Lamellarity Index |
|---|---|---|---|
| Comparative Mica A | 33 | 5.5 | 5.0 |
| Comparative Mica B | 14.4 | 3.3 | 3.4 |
| Sericite | 15.6 | 4.4 | 2.5 |
| Mica A | 18.4 | 5.9 | 2.1 |
| Mica B | 11.8 | 3.8 | 2.1 |
| Mica C | 16.4 | 6 | 1.7 |
| Mica D | 10.3 | 4.3 | 1.4 |

The composition of the pressed powders is shown in Table 2 below.

**Table 2.**

| Material | Function | Wt % |
|---|---|---|
| Mineral | Cohesive agent | 75.8 |
| Zinc Stearate | Other cohesive agent | 10.0 |
| Perfluorooctyl Triethoxysilane & CI77891 | Pigment | 3.2 |
| Perfluorooctyl Triethoxysilane & CI77499 | Pigment | 0.4 |
| Perfluorooctyl Triethoxysilane & CI77492 | Pigment | 0.8 |
| Perfluorooctyl Triethoxysilane & CI77491 | Pigment | 0.6 |
| Isocetyl Stearoyl Stearate | Binder | 9.0 |
| Geogard 111S | Preservative | 0.2 |

The pressed powders were made using the following procedure.

The powder cohesive agents were weighed. The pigments were pre-ground using a blender and added to the powder cohesive agents, and mixed with a SpeedMixer until homogenised. The liquid binder and preservative were added drop by drop and blended with the mixture three times using a SpeedMixer until homogenised. Approximately 11 g of the mixture is weighed in a container and the powder mixture is pressed for 5 seconds at 4 bars.

Cohesion was determined by a drop test. For each formulation, three dishes are dropped in an upright position inside a cylinder onto a glass plaque from a distance of 30 cm. This is repeated until the pressed powder formulation cracks. The number of drops taken to crack the pressed powder is recorded.

The results are shown in Table 3 below.

Table 3.

| Mineral | $d_{50laser}$ (µm) | $d_{50sedi}$ (µm) | Lamellarity Index | Number of Drops |
|---|---|---|---|---|
| Comparative Mica A | 33 | 5.5 | 5.0 | 2 |
| Comparative Mica B | 14.4 | 3.3 | 3.4 | 3 |
| Sericite | 15.6 | 4.4 | 2.5 | 12 |
| Mica A | 18.4 | 5.9 | 2.1 | 5 |
| Mica B | 11.8 | 3.8 | 2.1 | 6 |
| Mica C | 16.4 | 6 | 1.7 | 7 |
| Mica D | 10.3 | 4.3 | 1.4 | 10 |

It was surprisingly found that decreasing the lamellarity index of mica increases the number of drops the pressed powder can withstand before cracking. In particular, mica having a lamellarity index of less than about 3.0 provides a crack resistance that is commercially acceptable for a cosmetic composition.

Sensorial, optical and pick-up properties of the pressed powders was also determined and it was found that properties such as ease of application, homogeneity, softness (pick-up), adherence, and pick-up were approximately the same as the standard mica benchmark.

*Example 2*

Mica C was used to make a pressed powder comprising a higher amount of mica. The pressed powder formulation shown in Table 4 below was successfully made.

Table 4.

| Material | Function | Wt % |
|---|---|---|
| Sodium Dehydroacetate | Preservative | 0.2 |
| Zinc Stearate | Cohesive agent | 2.0 |
| Mica | Cohesive agent | 82.8 |
| Isostearyl Isostearate | Binder | 7.0 |
| Octyldodecanol & Octyldodecyl xyloside | Binder | 8.0 |

The foregoing broadly describes certain embodiments of the present invention without limitation. Variations and modifications as will be readily apparent to those skilled in the art are intended to be within the scope of the present invention as defined in and by the appended claims.

The following numbered paragraphs may defined particular embodiments of the present invention:

1. A pressed powder comprising mica having a lamellarity index of less than about 3.0, wherein the mica is present in the pressed powder in an amount equal to or greater than about 50 wt% and/or equal to or less than about 90 wt% of the mica.

2. The pressed powder of paragraph 1, wherein the pressed powder may further comprise one or more binder(s).

3. The pressed powder of paragraph 2, wherein the binder may constitute up to about 30.0 % by weight of the pressed powder, for example, from about 1.0 % to about 30.0 % by weight, or from about 1.0 % to about 25.0 % by weight, or from about 2.0 % to about 20.0 % by weight, or from about 2.0 % to about 15.0 % by weight, or from about 2.0 to about 10.0 % by weight, or from about 2.0 to about 7.5 % by weight of the pressed powder.

4. The pressed powder according to any one of paragraphs 1 to 3, wherein the mica has a BET surface area equal to or greater than about 3 $m^2/g$ for example, the mica has a BET surface area equal to or greater than about 4 $m^2/g$ or equal to or greater than about 5 $m^2/g$ or equal to or greater than about 6 $m^2/g$ or equal to or greater than about 7 $m^2/g$.

5. The pressed powder according to any one of paragraphs 1 to 3, wherein the mica has a BET surface area equal to or less than about 15 $m^2/g$. More preferably, the mica has a BET surface area equal to or less than about 14 $m^2/g$ or equal to or less than about 13 $m^2/g$ or equal to or less than about 12 $m^2/g$ or equal to or less than about 11 $m^2/g$ or equal to or less than about 10 $m^2/g$.

6. The pressed powder according to any one of paragraphs 1 to 3, wherein the mica has a BET surface area ranging from about 3 $m^2/g$ to about 15 $m^2/g$ or from about 5 $m^2/g$ to about 12 $m^2/g$ or from about 6 $m^2/g$ to about 10 $m^2/g$.

7. The pressed powder according to any preceding paragraph wherein the pressed powder comprises equal to or less than about 5.0 wt% moisture, for example equal to or less than about 4.5 wt% or equal to or less than about 4.0 wt% or equal to or less than about 3.5 wt% or equal to or less than about 3.0 wt% or equal to or less than about 2.5 wt% or equal to or less than about 2.0 wt% or equal to or less than about 1.5 wt% or equal to or less than about 1.0 wt% moisture.

8. Use of mica having a lamellarity index of less than about 3.0 as a cohesion enhancer in a pressed powder.

9. The use of a mica according to paragraph 8, wherein the mica is present in the pressed powder in an amount equal to or greater than about 50 wt% and/or equal to or less than about 90 wt% of the mica.

10. The use of a mica according to paragraph 8 or 9, wherein the pressed powder may further comprise one or more binder(s).

11. The use of a mica according to paragraph 10, wherein the binder may constitute up to about 30.0 % by weight of the pressed powder, for example, from about 1.0 % to about 30.0 % by weight, or from about 1.0 % to about 25.0 % by weight, or from about 2.0 % to about 20.0 % by weight, or from about 2.0 % to about 15.0 % by weight, or from about 2.0 to about 10.0 % by weight, or from about 2.0 to about 7.5 % by weight of the pressed powder.

12. The use of a mica according to any one of paragraphs 8 to 11, wherein the mica has a BET surface area equal to or greater than about 3 $m^2/g$ for example, the mica has a BET surface area equal to or greater than about 4 $m^2/g$ or equal to or greater than about 5 $m^2/g$ or equal to or greater than about 6 $m^2/g$ or equal to or greater than about 7 $m^2/g$.

13. The use of a mica according to any one of paragraphs 8 to 12, wherein the mica has a BET surface area equal to or less than about 15 $m^2/g$. More preferably, the mica has a BET surface area equal to or less than about 14 $m^2/g$ or equal to or less than about 13 $m^2/g$ or equal to or less than about 12 $m^2/g$ or equal to or less than about 11 $m^2/g$ or equal to or less than about 10 $m^2/g$.

14. The use of a mica according to any one of paragraphs 8 to 12, wherein the mica has a BET surface area ranging from about 3 $m^2/g$ to about 15 $m^2/g$ or from about 5 $m^2/g$ to about 12 $m^2/g$ or from about 6 $m^2/g$ to about 10 $m^2/g$.

15. The use of a mica according to any preceding paragraph wherein the pressed powder comprises equal to or less than about 5.0 wt% moisture, for example equal to or less than about 4.5 wt% or equal to or less than about 4.0 wt% or equal to or less than about 3.5 wt% or equal to or less than about 3.0 wt% or equal to or less than about 2.5 wt% or equal to or less than about 2.0 wt% or equal to or less than about 1.5 wt% or equal to or less than about 1.0 wt% moisture.

**Claims**

1. A pressed powder comprising mica having a lamellarity index of less than 3.0, wherein the mica is present in the pressed powder in an amount equal to or greater than 50 wt% and/or equal to or less than 90 wt% of the mica.

2. The pressed powder of claim 1, wherein the mica has a lamellarity index equal to or greater than 0.5, for example greater than 1.0.

3. The pressed powder of claim 1 or 2, wherein the mica comprises at least 70 wt% muscovite, for example at least 85 wt% muscovite.

4. The pressed powder of any preceding claim, wherein the mica has a $d_{50laser}$ equal to or less than 40.0 $\mu$m and/or equal to or greater than 3.0 $\mu$m, for example equal to or less than 20.0 $\mu$m and/or equal to or greater than 8.0 $\mu$m.

5. The pressed powder of preceding claim, wherein the mica has a $d_{50seidigraph}$ equal to or less than 20.0 $\mu$m and/or equal to or greater than 0.5 $\mu$m, for example equal to or less than 8.0 $\mu$m and/or equal to or greater than 2.0 $\mu$m.

6. The pressed powder of any preceding claim, wherein the pressed powder comprises equal to or less than 10 wt% of a cohesive agent other than the mica.

7. The pressed powder of any preceding claim, wherein the mica is uncoated.

8. Use of mica having a lamellarity index of less than 3.0 as a cohesion enhancer in a pressed powder.

9. The use of claim 8, wherein the mica has a lamellarity index equal to or greater than 0.5, for example greater than 1.0.

10. The use of claims 8 or 9, wherein the mica comprises at least 70 wt% muscovite, for example at least 85 wt% muscovite.

11. The use of claims 8 to 10, wherein the mica is uncoated.

12. The use of claims 8 to 11, wherein the mica has a $d_{50laser}$ equal to or less than 40.0 $\mu$m and/or equal to or greater than 3.0 $\mu$m, for example equal to or less than 20.0 $\mu$m and/or equal to or greater than 6.0 $\mu$m.

13. The use of any claims 8 to 12, wherein the mica has a $d_{50sedigraph}$ equal to or less than 20.0 $\mu$m and/or equal to or greater than 0.5 $\mu$m, for example equal to or less than 8.0 $\mu$m and/or equal to or greater than 2.0 $\mu$m.

14. The use of claims 8 to 13, wherein the pressed powder comprises equal to or greater than 50 wt% and/or equal to or less than 90 wt% of the mica.

15. The use of claims 8 to 14, wherein the pressed powder comprises equal to or less than 10 wt% of a cohesive agent other than the mica.

**Patentansprüche**

1. Gepresstes Puder, umfassend Glimmer mit einem Lamellaritätsindex von weniger als 3,0, wobei der Glimmer in dem gepressten Puder vorhanden in einer Menge gleich oder größer als 50 Gew.-% und/oder gleich oder kleiner als 90 Gew.-% des Glimmers ist.

2. Gepresstes Puder nach Anspruch 1, wobei der Glimmer einen Lamellaritätsindex gleich oder größer als 0,5, beispielsweise größer als 1,0, aufweist.

3. Gepresstes Puder nach Anspruch 1 oder 2, wobei der Glimmer mindestens 70 Gew.-% Muskovit, beispielsweise mindestens 85 Gew.-% Muskovit, umfasst.

4. Gepresstes Puder nach einem vorhergehenden Anspruch, wobei der Glimmer einen $d_{50Laser}$ gleich oder kleiner als 40,0 $\mu$m und/oder gleich oder größer als 3,0 $\mu$m, beispielsweise gleich oder kleiner als 20,0 $\mu$m und/oder gleich oder größer als 8,0 $\mu$m, aufweist.

5. Gepresstes Puder nach vorhergehendem Anspruch, wobei der Glimmer einen $d_{50Sedigraph}$ gleich oder kleiner als 20,0 $\mu$m und/oder gleich oder größer als 0,5 $\mu$m, beispielsweise gleich oder kleiner als 8,0 $\mu$m und/oder gleich oder größer als 2,0 $\mu$m, aufweist.

6. Gepresstes Puder nach einem vorhergehenden Anspruch, wobei das gepresste Puder gleich oder kleiner als 10 Gew.-% eines Bindemittels außer dem Glimmer umfasst.

7. Gepresstes Puder nach einem vorhergehenden Anspruch, wobei der Glimmer unbeschichtet ist.

**8.** Verwendung von Glimmer mit einem Lamellaritätsindex von weniger als 3,0 als Kohäsionsverstärker in einem gepressten Puder.

**9.** Verwendung nach Anspruch 8, wobei der Glimmer einen Lamellaritätsindex gleich oder größer als 0,5, beispielsweise größer als 1,0, aufweist.

**10.** Verwendung nach Anspruch 8 oder 9, wobei der Glimmer mindestens 70 Gew.-% Muskovit, beispielsweise mindestens 85 Gew.-% Muskovit, umfasst.

**11.** Verwendung nach den Ansprüchen 8 bis 10, wobei der Glimmer unbeschichtet ist.

**12.** Verwendung nach den Ansprüchen 8 bis 11, wobei der Glimmer einen $d_{50Laser}$ gleich oder kleiner als 40,0 $\mu$m und/oder gleich oder größer als 3,0 $\mu$m, beispielsweise gleich oder kleiner als 20,0 $\mu$m und/oder gleich oder größer als 6,0 $\mu$m, aufweist.

**13.** Verwendung nach einem der Ansprüche 8 bis 12, wobei der Glimmer einen $d_{50Sedigraph}$ gleich oder kleiner als 20,0 $\mu$m und/oder gleich oder größer als 0,5 $\mu$m, beispielsweise gleich oder kleiner als 8,0 $\mu$m und/oder gleich oder größer als 2,0 $\mu$m, aufweist.

**14.** Verwendung nach den Ansprüchen 8 bis 13, wobei das gepresste Puder gleich oder größer als 50 Gew.-% und/oder gleich oder kleiner als 90 Gew.-% des Glimmers umfasst.

**15.** Verwendung nach den Ansprüchen 8 bis 14, wobei das gepresste Puder gleich oder kleiner als 10 Gew.-% eines Bindemittels außer dem Glimmer umfasst.

**Revendications**

**1.** Poudre comprimée comprenant du mica présentant un indice de lamellarité inférieur à 3,0, dans laquelle le mica est présent dans la poudre comprimée à une teneur égale ou supérieure à 50 % en poids et/ou égale ou inférieure à 90 % en poids.

**2.** Poudre comprimée selon la revendication 1, dans laquelle le mica présente un indice de lamellarité égal ou supérieur à 0,5, par exemple supérieur à 1,0.

**3.** Poudre comprimée selon la revendication 1 ou 2, dans laquelle le mica comprend une teneur en muscovite d'au moins 70 % en poids, par exemple au moins 85 % en poids.

**4.** Poudre comprimée selon une quelconque revendication précédente, dans laquelle le mica présente un $d_{50laser}$ égal ou inférieur à 40,0 $\mu$m et/ou égal ou supérieur à 3,0 $\mu$m, par exemple égal ou inférieur à 20,0 $\mu$m et/ou égal ou supérieur à 8,0 $\mu$m.

**5.** Poudre comprimée selon une revendication précédente, dans laquelle le mica présente un $d_{50sedigraph}$ égal ou inférieur à 20,0 $\mu$m et/ou égal ou supérieur à 0,5 $\mu$m, par exemple égal ou inférieur à 8,0 $\mu$m et/ou égal ou supérieur à 2,0 $\mu$m.

**6.** Poudre comprimée selon une quelconque revendication précédente, dans laquelle la poudre comprimée comprend une teneur en agent cohésif autre que le mica égale ou inférieure à 10 % en poids.

**7.** Poudre comprimée selon une quelconque revendication précédente, dans laquelle le mica est non revêtu.

**8.** Utilisation de mica présentant un indice de lamellarité inférieur à 3,0 comme agent de cohésion dans une poudre comprimée.

**9.** Utilisation selon la revendication 8, dans laquelle le mica présente un indice de lamellarité égal ou supérieur à 0,5, par exemple supérieur à 1,0.

**10.** Utilisation selon les revendications 8 ou 9, dans laquelle le mica comprend une teneur en muscovite d'au moins 70 % en poids, par exemple au moins 85 % en poids.

11. Utilisation selon les revendications 8 à 10, dans laquelle le mica est non revêtu.

12. Utilisation selon les revendications 8 à 11, dans laquelle le mica présente un $d_{50laser}$ égal ou inférieur à 40,0 $\mu$m et/ou égal ou supérieur à 3,0 $\mu$m, par exemple égal ou inférieur à 20,0 $\mu$m et/ou égal ou supérieur à 6,0 $\mu$m.

13. Utilisation selon les revendications 8 à 12, dans laquelle le mica présente un $d_{50sedigraph}$ égal ou inférieur à 20,0 $\mu$m et/ou égal ou supérieur à 0,5 $\mu$m, par exemple égal ou inférieur à 8,0 $\mu$m et/ou égal ou supérieur à 2,0 $\mu$m.

14. Utilisation selon les revendications 8 à 13, dans laquelle la poudre comprimée comprend une teneur en mica égale ou supérieure à 50 % en poids et/ou égale ou inférieure à 90 % en poids.

15. Utilisation selon les revendications 8 à 14, dans laquelle la poudre comprimée comprend une teneur en agent cohésif autre que le mica égale ou inférieure à 10 % en poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016146708 A1

**Non-patent literature cited in the description**

- **G. BAUDET** ; **J. P. RONA**. *Ind. Min. Mines et Carr. Les techn. June*, July 1990, 55-61
- *CHEMICAL ABSTRACTS*, 68915-31-1
- *CHEMICAL ABSTRACTS*, 9002-93-1